# EUROPÄISCHE PATENTSCHRIFT

(11) **EP 0 468 026 B1**
(45) Veröffentlichungstag und Bekanntmachung des Hinweises auf die Patenterteilung: **17.11.1994**
(21) Anmeldenummer: 91903842.2
(22) Anmeldetag: 11.02.1991
(51) Int. Cl.: C03C 4/00, C03C 10/16, A61L 27/00

(54) **APATITGLASKERAMIK**
APATITE GLASS CERAMIC
VITROCERAMIQUE APATITE

(30) Priorität: 12.02.1990 DD 337765
(43) Veröffentlichungstag der Anmeldung: 29.01.1992
(73) Patentinhaber: THERA Patent GmbH & Co. KG Gesellschaft für industrielle Schutzrechte, D-82229 Seefeld (DE)
(72) Erfinder: JANA, Carsten, D-6908 Jena-Winzerla (DE); HÖLAND, Wolfram, D-6908 Jena-Winzerla (DE); VOGEL, Werner, D-6900 Jena (DE)
(74) Vertreter: Abitz, Walter, Dr.-Ing.
(86) Internationale Anmeldenummer: EP9100265
(87) Internationale Veröffentlichungsnummer: WO9112212

(56) Entgegenhaltungen:
- EP-A- 0 023 013
- EP-A- 0 386 525
- US-A- 4 643 982

## Beschreibung

Die Erfindung betrifft eine Apatitglaskeramik, die aufgrund ihrer Eigenschaften als Biomaterial in der Medizin und insbesondere in der Stomatologie als anorganische Komponente für Glasionomerzemente eingesetzt werden kann.

Apatithaltige Glaskeramiken und Apatitsinterkeramiken sind bekannt und werden z.B. in der DD 219 017 und in der DD 245 864 beschrieben. Derartige Biowerkstoffe bilden direkte Bindungen zu lebenden Knochen aus.

Als anorganische Komponente für Glasionomerzemente werden jedoch spezielle Anforderungen an eine Glaskeramik gestellt. Apatithaltige Glaskeramiken für Glasionomerzemente sind bisher nicht entwickelt worden. Glasionomerzemente entstehen durch das Vermischen von Fluoralumosilikatglaspulver und polymeren Carbonsäuren. Glasionomerzemente werden in der Stomatologie für geeignete Indikationen wie Unterfüllung, Stumpfaufbau und Befestigung verwendet.

Eine alleinige Kombination von Fluoralumosilikatglaspulvern mit Polyacrylsäure ergibt Glasionomerzemente mit schlechten Verarbeitungseigenschaften. Der Glasionomerzement benötigt zu viel Zeit zum vollständigen Abbinden, so daß dessen Oberfläche durch Speichelkontakt im Munde des Patienten brüchig wird. Zur Überwindung dieser Nachteile offenbart beispielsweise die japanische Patentschrift 52 (1977) 101 893 eine wäßrige Polyacrylsäure- bzw. Acrylpolymer-Lösung, die eine oder mehrere mehrfach basische Carbonsäuren enthält. Durch Verwendung dieser Flüssigkeit wurden geringere Abbindezeiten und höhere mechanische Festigkeiten erzielt. Die US-Patentschrift 4 360 605 offenbart eine Härterflüssigkeit, die neben einem Acrylsäurecopolymer Weinsäure enthält. Dabei wurde festgestellt, daß damit das Verhältnis Verarbeitungszeit zu Abbindezeit weiter verbessert wurde. Bei Glasionomerzementen sollte die Verarbeitungszeit lang und die Abbindezeit möglichst kurz sein. In dieser Hinsicht erfüllen diese Glasionomerzemente noch nicht die Anforderungen des Anwenders.

Der Abbindevorgang bei Glasionomerzementen ist durch die Bildung von Calcium- und Aluminiumpolycarboxylaten gekennzeichnet. Im ersten Reaktionsschritt bildet sich ein noch relativ wasserempfindliches Calciumpolycarboxylat, erst die Bildung von Aluminiumpolycarboxylat, die aufgrund des höheren Ordnungsgrades und der geringeren Wanderungsgeschwindigkeit der höher geladenen Aluminiumionen zeitlich später erfolgt, führt zum stabilen Zementsystem. Fluoralumosilikatglaspulver des Systems SiO₂-Al₂O₃-CaO-P₂O₅-Na₂O-F⁻ nach der US-Patentschrift 4 376 835 haben den Nachteil, daß Biegefestigkeit und Haftung an der Zahnhartsubstanz ungenügend sind. Nach Wilson und Mclean [Glass Ionomer Cement, Quintessenz-Verlag Berlin (West) 1988, 28] kann die Funktion der Calcium-Ionen im Abbindeprozeß durch Strontium-Ionen übernommen werden. Strontiumhaltige Fluoralumosilikatglaspulver nach DE-A 3 804 469 zeigen zwar verbesserte Eigenschaften hinsichtlich Löslichkeit und Röntgensichtbarkeit, weisen aber ungenügende Biegefestigkeiten und Haftung an der Zahnhartsubstanz auf.

Wilson und Mitarbeiter (Ind. Eng. Chem. Prod. Res. Dev. 19, 1980, 263-270) untersuchten Gläser, bei denen in einem Nebenprozeß CaF₂-Kristalle entstanden waren. Es wurde festgestellt, daß diese Gläser eine geringere mechanische Festigkeit als die entsprechenden klaren Gläser besitzen.

Aufgabe der Erfindung ist es, eine apatithaltige Glaskeramik insbesondere für Glasionomerzement zu entwickeln, die die Handhabbarkeit und Haftung gegenüber bekannten technischen Lösungen von Glasionomerzementen verbessert.

Die Aufgabe wird erfindungsgemäß dadurch gelöst, daß die Apatitglaskeramik die Zusammensetzung

| | |
|---|---|
| SiO₂ | 15 - 40 Ma.-% |
| Al₂O₃ | 15 - 35 Ma.-% |
| CaO | 12 - 30 Ma.-% |
| P₂O₅ | 6 - 25 Ma.-% |
| F⁻ | 2,5 - 15 Ma.-% |
| R₂O | 0 - 18 Ma.-% |

auf der Basis der Oxide berechnet, besitzt, wobei R₂O Alkalioxid - bevorzugt Na₂O - ist, und neben der Glasphase die Kristallphase Apatit enthält. Vorzugsweise ist Apatit als Hauptkristallphase enthalten. Daneben können z.B. Sillimanit, Cristobalit und Fluorit enthalten sein. Sillimanit und Cristobalit können auch die Hauptkristallphase bilden. Zusatzkomponenten wie z.B. SrO, FeO, Fe₂O₃, La₂O₃, TiO₂ können bis zu 25 Ma.-% einzeln oder im Gemisch ebenfalls enthalten sein. Die Zusatzkomponenten werden zugegeben, um die Glaskeramik hinsichtlich solcher Eigenschaften wie Färbung, Röntgensichtbarkeit, Opazität, Transluzenz vorteilhaft zu beeinflussen. Vorzugsweise ist die Zusammensetzung wie folgt:

| | |
|---|---|
| SiO₂ | 15 - 40 Ma.-% |
| Al₂O₃ | 15 - 35 Ma.-% |
| CaO | 12 - 30 Ma.-% |
| P₂O₅ | 8 - 25 Ma.-% |
| F⁻ | 3 - 12 Ma.-% |
| R₂O | 3,5 - 16 Ma.-% . |

Besonders bevorzugt ist die Zusammensetzung wie folgt:

| | |
|---|---|
| SiO₂ | 15 - 40 Ma.-% |
| Al₂O₃ | 15 - 35 Ma.-% |
| CaO | 12 - 30 Ma.-% |
| P₂O₅ | 8 - 25 Ma.-% |
| F⁻ | 4 - 11 Ma.-% |
| R₂O | 6 - 10 Ma.-% |

Sofern R₂O K₂O bedeutet, enthält die Apatitglaskeramik vorzugsweise maximal 8 Ma.-%, insbesondere maximal 5 Ma.-% K₂O.

Die Kristallphase Apatit erhöht die mechanische Festigkeit und stimuliert das Knochenwachstum. Dabei sind in der erfindungsgemäßen Apatitglaskeramik vorzugsweise Fluorapatit, aber auch Hydroxylapatit oder gemischter Fluor-Hydroxylapatit enthalten. In diese Apatitphasen können auch Natriumionen und/oder Kaliumionen und/oder Aluminiumionen und/oder Strontiumionen eingebaut sein.

Das erfindungsgemäße Glaskeramikmaterial wird aus einem Ausgangsglas, das bei 1450°C bis 1600°C erschmolzen wird, hergestellt. Dabei wurde überraschend festgestellt, daß die Gläser tröpfchenförmige Entmischungsbezirke, oftmals mit Mehrfachentmischung, besitzen und bei nachfolgender Temperaturbehandlung Apatitkristalle ausscheiden. Das ist umso erstaunlicher, da in anderen Zusammensetzungsbereichen eine gesteuerte Ausscheidung von Apatitkristallen über eine Phasentrennung amorph/amorph nicht realisierbar ist.

Das Ausgangsglas wird bis unterhalb der Transformationstemperatur abgekühlt oder direkt aus der Schmelze einer gesteuerten Kristallisation unterworfen. Die gesteuerte Kristallisation erfolgt durch thermische Behandlung der Gläser einstufig im Temperaturbereich 700°C bis 1150°C. Die Steuerung des Kristallisationsprozesses des Ausgangsglases zur erfindungsgemäßen Glaskeramik erfolgt durch eine gezielte Beeinflussung der Phasentrennung der Gläser.

Aus Ca²⁺-P₂O₅-F⁻-reichen tröpfchenförmigen primären Entmischungsbezirken erfolgt die Ausscheidung von Apatitkristallen im Glas. Durch Wahl der chemischen Zusammensetzung, der Schmelzbedingungen und der thermischen Behandlung lassen sich Volumenanteil, Anzahl und Größe dieser Entmischungsbezirke einstellen, damit ist Volumenanteil und Kristallgröße der Apatitphase steuerbar. Erfindungsgemäß besitzen die Apatitkristalle der Apatitglaskeramik vorzugsweise eine Kristallgröße von 0,2 bis 3 µm.

Für den Einsatz der erfindungsgemäßen Apatitglaskeramik als anorganische Komponente in Glasionomerzement wird ein Pulver der Glaskeramik verwendet, wobei die Pulverkörner vorzugsweise eine durchschnittliche Korngröße (Gewichtsmittel) von wenigstens 1 µm und bevorzugt mindestens 3 µm aufweisen. Die durchschnittliche Korngröße (Gewichtsmittel) beträgt 1 - 20 µm, bevorzugt 3 - 15 µm und besonders bevorzugt 3 - 10 µm. Die Teilchen haben eine maximale Korngröße von 150 µm, vorzugsweise 100 µm, besonders 60 µm.

Der Glasionomerzement wird hergestellt durch Aushärten dieses Glaskeramikpulvers und einer Polysäure in Gegenwart von Wasser und gegebenenfalls anderer üblicher Zusätze, wie z.B. Weinsäure. Die so erhaltenen Pulver werden dann gegebenenfalls einer Oberflächenbehandlung gemäß der europäischen Patentschrift 0 023 013 unterzogen. Hierzu werden die Glaspulver mit Säure oberflächlich behandelt, vorzugsweise bei Raumtemperatur. Dabei werden saure Gruppen enthaltende Substanzen eingesetzt, z.B. Salzsäure, Schwefelsäure, Salpetersäure, Essigsäure, Propionsäure oder Perchlorsäure, die lösliche Calciumsalze bzw. Strontiumsalze bilden. Die Säuren werden in einer Konzentration von 0,01 bis 10 Gew.-%, vorzugsweise von 0,05 bis 3 Gew.-% eingesetzt. Nach der entsprechenden Reaktionszeit werden die Pulver von der Lösung separiert und gründlich ausgewaschen, so daß sich praktisch keine löslichen Calcium- oder Strontiumsalze mehr auf der Oberfläche der Pulverteilchen befinden.

Als Polysäuren können die im Zusammenhang mit Glasionomerzement bekannten Polysäuren verwendet werden, z.B. Polymaleinsäure, Polyacrylsäure, Polyitaconsäure sowie Gemisch davon oder Copolymere, insbesondere die aus der EP-B-0 024 056 bekannten Maleinsäure-Acrylsäure-Copolymere und/oder Acrylsäure-Itaconsäure-Copolymere. Das mittlere Molekulargewicht der erfindungsgemäß einzusetzenden Polycarbonsäuren beträgt mehr als 500. Vorteilhaft ist ein mittleres Molekulargewicht von 1.000 bis 20.000, besonders bevorzugt sind 3.000 bis 10.000. Die Polycarbonsäure wird vorzugsweise in Konzentrationen von 5 bis 50 Gew.-%, bezogen auf das Gewicht der Apatitglaskeramik, eingesetzt. Als Polysäure geeignet sind auch Polyphosphonsäuren, z.B. Polyvinylphosphonsäure. Diese Polyphosphonsäuren können die oben genannten Polycarbonsäuren ganz oder teilweise ersetzen.

Es wurde überraschend festgestellt, daß aus Apatitglaskeramikpulver hergestellte Glasionomerzemente bessere Verarbeitungseigenschaften, rasche Erhärtung und eine erhöhte mechanische Festigkeit besitzen. Das Vorhandensein der Apatit-Kristallphase bewirkt eine bessere Haftung des Glasionomerzementes an der Zahnhartsubstanz.

### Ausführungsbeispiele

Ein Überblick über die Zusammensetzungen in Ma.-% der erfindungsgemäßen Apatitglaskeramik bzw. deren Ausgangsgläser wird in Tabelle 1 gegeben. Die Tabelle 2 enthält ausgewählte Beispiele, die den Zusammenhang zwischen chemischer Zusammensetzung, thermischer Behandlung der Ausgangsgläser und Kristallphase, Kristallgröße aufzeigen.

**Tabelle 1**

| Zusammensetzungen der Apatitglaskeramik bzw. deren Ausgangsgläsern | | | | | | | | | | |
|---|---|---|---|---|---|---|---|---|---|---|
| Beisp. Nr. | SiO₂ | Al₂O₃ | CaO | P₂O₅ | Na₂O | F⁻ | SrO | Fe₂O₃ | K₂O | La₂O₃ |
| 1 | 15,2 | 34,6 | 24,3 | 15,7 | 6,1 | 4,1 | - | - | - | - |
| 2 | 15,3 | 15,2 | 24,5 | 24,1 | 15,7 | 5,2 | - | - | - | - |
| 3 | 39,8 | 15,3 | 12,7 | 8,2 | 14,0 | 10,0 | - | - | - | - |
| 4 | 22,0 | 26,4 | 20,5 | 15,6 | 6,2 | 9,3 | - | - | - | - |
| 5 | 26,3 | 20,2 | 28,1 | 9,4 | 6,1 | 9,9 | - | - | - | - |
| 6 | 23,9 | 24,8 | 14,3 | 17,7 | 7,3 | 7,1 | 4,9 | - | - | - |
| 7 | 21,0 | 17,9 | 23,9 | 12,3 | 11,9 | 9,0 | 3,6 | 0,4 | - | - |
| 8 | 19,8 | 23,6 | 25,7 | 15,8 | 7,7 | 7,4 | - | - | - | - |
| 9 | 22,1 | 22,8 | 19,1 | 16,0 | 9,6 | 6,5 | - | - | 3,9 | - |
| 10 | 31,4 | 28,5 | 13,0 | 8,5 | 9,6 | 9,0 | - | - | - | - |
| 11 | 22,0 | 26,8 | 20,2 | 15,8 | 8,2 | 7,0 | - | - | - | - |
| 12 | 27,3 | 24,7 | 18,7 | 12,9 | 8,0 | 8,4 | - | - | - | - |
| 13 | 23,4 | 15,9 | 19,3 | 14,6 | 9,4 | 9,4 | - | - | - | 8,0 |
| 14 | 24,9 | 25,8 | 20,0 | 18,4 | 3,5 | 7,4 | - | - | - | - |
| 15 | 25,8 | 26,7 | 20,7 | 19,1 | - | 7,7 | - | - | - | - |
| 16 | 24,5 | 16,8 | 16,4 | 18,3 | 16,2 | 7,8 | - | - | - | - |
| 17 | 23,4 | 23,9 | 19,3 | 14,6 | 9,4 | 9,4 | - | - | - | - |

**Tabelle 2**

| Thermische Behandlung der Ausgangsgläser und daraus resultierende Kristallphasen und Kristallitgrößen | | | |
|---|---|---|---|
| Beisp. Nr. | Thermische Behandlung | Kristallphase | Kristallgröße |
| 8 | 950°C / 1 h | Apatit | 0,2 - 0,6 µm |
| 8 | 1050°C / 1 h | Apatit | 0,5 - 1 µm |
| 8 | 1150°C / 1 h | Apatit | 1,5 - 4 µm |
| 9 | 900°C / 2 h | Apatit | 0,3 - 0,7 µm |
| 10 | 950°C / 2 h | Apatit | 0,7 - 2 µm |
| 11 | 800°C / 1 h | Apatit | 0,2 - 0,3 µm |
| 14 | 920°C / 2 h | Apatit (Hauptphase) Cristobalit, Sillimanit (Nebenphasen) | < 2 µm |
| 15 | 920°C / 2 h | Cristobalit, Sillimanit (Hauptphasen) | <2 µm |
| | | Apatit (Nebenphase) | 2 µm |
| 16 | 910°C / 2 h | Apatit | 0,25 - 0,7 µm |
| 17 | 910°C / 2 h | Apatit | 0,25 - 0,35 µm |

### Beispiel 18

Die nach Tabelle 2 thermisch behandelte Glaskeramik von Beispiel 16 wurde zu einem feinen Pulver der mittleren Korngröße 6,2 µm (Gewichtsmittel) vermahlen; die maximale Korngröße betrug 40 µm.

1 g dieses Pulvers wurde mit 0,33 g einer wäßrigen Polycarbonsäurelösung der folgenden Zusammensetzung vermischt:
50 Gewichtsteile Wasser
40 Gewichtsteile eines Copolymeren aus Acrylsäure und Maleinsäure (1:1, mittleres Molgewicht 13.000)
10 Gewichtsteile Weinsäure
Die erhaltene Zementmischung war bei 33°C Raumtemperatur 3 min 15 sec plastisch verformbar (Verarbeitungszeit) und bereits nach 3 min 20 sec erhärtet (Aushärtezeit), jeweils gerechnet vom Beginn des Anmischens.

Nach 24-stündiger Aushärtung bei 36°C betrug die Biegefestigkeit 39,7 MPa.

### Beipiel 19

Die nach Tabelle 2 thermisch behandelte Glaskeramik von Beispiel 17 wurde zu einem feinen Pulver der mittleren Korngröße 7,8 µm (Gewichtsmittel) vermahlen; die maximale Korngröße betrug 45 µm.

1 g dieses Pulvers wurde mit 0,33 g der wäßrigen Polycarbonsäurelösung von Beispiel 18 vermischt.

Die erhaltene Zementmischung hatte bei 23°C Raumtemperatur 2 min 40 sec Verarbeitungszeit und nur 3 min 55 sec Aushärtezeit, jeweils gerechnet vom Beginn des Anmsichens.

Nach 24-stündiger Aushärtung bei 36°C betrug die Biefefestigkeit 34,9 MPa.

## Patentansprüche

1. Apatitglaskeramik, dadurch gekennzeichnet, daß sie die Zusammensetzung
| | |
|---|---|
| SiO₂ | 15 - 40 Ma.-% |
| Al₂O₃ | 15 - 35 Ma.-% |
| CaO | 12 - 30 Ma.-% |
| P₂O₅ | 6 - 25 Ma.-% |
| F⁻ | 2,5 - 15 Ma.-% |
| R₂O | 0 - 18 Ma.-% |
auf der Basis der Oxide berechnet, besitzt, wobei R₂O Alkalioxid ist und die Kristallphase Apatit enthält.

2. Apatitglaskeramik nach Anspruch 1, dadurch gekennzeichnet, daß die Hauptkristallphase durch Apatit gebildet wird.

3. Apatitglaskeramik nach den Ansprüchen 1 oder 2, dadurch gekennzeichnet, daß sie die Zusammensetzung
| | |
|---|---|
| SiO₂ | 15 - 40 Ma.-% |
| Al₂O₃ | 15 - 35 Ma.-% |
| CaO | 12 - 30 Ma.-% |
| P₂O₅ | 8 - 25 Ma.-% |
| F⁻ | 3 - 12 Ma.-% |
| R₂O | 3,5 - 16 Ma.-% |
hat.

4. Apatitglaskeramik nach den Ansprüchen 1 oder 2, dadurch gekennzeichnet, daß sie die Zusammensetzung
| | |
|---|---|
| SiO₂ | 15 - 40 Ma.-% |
| Al₂O₃ | 15 - 35 Ma.-% |
| CaO | 12 - 30 Ma.-% |
| P₂O₅ | 8 - 25 Ma.-% |
| F⁻ | 4 - 11 Ma.-% |
| R₂O | 6 - 10 Ma.-% |
hat.

5. Apatitglaskeramik nach den Ansprüchen 1 bis 4, dadurch gekennzeichnet, daß sie Zusatzkomponenten bis 25 Ma.-% einzeln oder im Gemisch enthält, mit deren Hilfe Röntgensichtbarkeit erreicht wird, vorzugsweise SrO, La₂O₃, oder eine Farbgebung bei der Glaskeramik erzielt wird, vorzugsweise FeO, Fe₂O₃, TiO₂.

6. Apatitglaskeramik nach den Ansprüchen 1 bis 5, dadurch gekennzeichnet, daß sie aus bei 1450°C bis 1600°C erschmolzenen, entmischten Ausgangsgläsern hergestellt worden ist.

7. Apatitglaskeramik nach den Ansprüchen 1 bis 6, dadurch gekennzeichnet, daß sie durch eine thermische Behandlung der entmischten Ausgangsgläser im Temperaturbereich 700°C bis 1150°C hergestellt worden ist.

8. Apatitglaskeramik nach den Ansprüchen 1 bis 7, dadurch gekennzeichnet, daß die Apatitkristalle vorzugsweise eine Kristallgröße von 0,2 µm bis 3 µm besitzen.

9. Apatitglaskeramik nach den Ansprüchen 1 bis 8, daudrch gekennzeichnet, daß Fluorapatit und/oder Hydroxylapatit und/oder gemischter Fluor-Hydroxylapatit die Hauptkristallphase Apatit darstellen.

10. Apatitglaskeramik nach den Ansprüchen 1 bis 9, dadurch gekennzeichnet, daß die Apatitphase
Aluminiumionen
und/oder Natriumionen
und/oder Kaliumionen
und/oder Strontiumionen
enthält.

11. Verwendung der Apatitglaskeramik nach den Ansprüchen 1 bis 10 als anorganische Komponente in Glasionomerzementen.

## Claims

1. Apatite glass ceramic, characterized in that it contains the composition
| | |
|---|---|
| SiO₂ | 15 - 40 wt-% |
| Al₂O₃ | 15 - 35 wt-% |
| CaO | 12 - 30 wt-% |
| P₂O₅ | 6 - 25 wt-% |
| F⁻ | 2.5 - 15 wt-% |
| R₂O | 0 - 18 wt-%, |
calculated on the basis of the oxides, R₂O being an alkali oxide and the crystal phase containing apatite.

2. Apatite glass ceramic according to claim 1, charaterized in that the main crystal phase is formed by apatite.

3. Apatite glass ceramic according to claims 1 or 2, characterized in that it has the composition
| | |
|---|---|
| SiO₂ | 15 - 40 wt-% |
| Al₂O₃ | 15 - 35 wt-% |
| CaO | 12 - 30 wt-% |
| P₂O₅ | 8 - 25 wt-% |
| F⁻ | 3 - 12 wt-% |
| R₂O | 3.5 - 16 wt-%. |

4. Apatite glass ceramic according to claims 1 or 2, characterized in that it has the composition
| | |
|---|---|
| SiO₂ | 15 - 40 wt-% |
| Al₂O₃ | 15 - 35 wt-% |
| CaO | 12 - 30 wt-% |
| P₂O₅ | 8 - 25 wt-% |
| F⁻ | 4 - 11 wt-% |
| R₂O | 6 - 10 wt-%. |

5. Apatite glass ceramic according to claims 1 to 4, characterized in that it contains up to 25 wt-% of additional components, individually or as a mixture, with the help of which X-ray visibility is achieved, preferably SrO, La₂O₃, or a coloration of the glass ceramic is achieved, preferably FeO, Fe₂O₃, TiO₂.

6. Apatite glass ceramic according to claims 1 to 5, characterized in that it has been prepared from demixed starting glasses melted at 1450°C to 1600°C.

7. Apatite glass ceramic according to claims 1 to 6, characterized in that it has been prepared by a thermal treatment of the demixed starting glasses in the temperature range 700°C to 1150°C.

8. Apatite glass ceramic according to claims 1 to 7, characterized in that the apatite crystals preferably have a crystal size of 0.2 µm to 3 µm.

9. Apatite glass ceramic according to claims 1 to 8, characterized in that fluorapatite and/or hydroxyl apatite and/or mixed fluor-hydroxyl apatite represent the main crystal phase apatite.

10. Apatite glass ceramic according to claims 1 to 9, charaterized in that the apatite phase contains
aluminium ions
and/or sodium ions
and/or potassium ions
and/or strontium ions.

11. Use of the apatite glass ceramic according to claims 1 to 10 as inorganic component in glass ionomer cements.

## Revendications

1. Vitrocéramique à base d'apatite, caractérisée en ce qu'elle a la composition suivante, sur la base des oxydes :
| | |
|---|---|
| SiO₂ | 15 - 40 % en masse |
| Al₂O₃ | 15 - 35 % en masse |
| CaO | 12 - 30 % en masse |
| P₂O₅ | 6 - 25 % en masse |
| F⁻ | 2,5 - 15 % en masse |
| R₂O | 0 - 18 % en masse |
où R₂O est l'oxyde d'un métal alcalin, la phase cristalline contenant de l'apatite.

2. Vitrocéramique à base d'apatite selon la revendication 1, caractérisée en ce que la phase cristalline principale est constituée d'apatite.

3. Vitrocéramique à base d'apatite selon les revendications 1 ou 2, caractérisée en ce qu elle a la composition suivante :
| | |
|---|---|
| SiO₂ | 15 - 40 % en masse |
| Al₂O₃ | 15 - 35 % en masse |
| CaO | 12 - 30 % en masse |
| P₂O₅ | 8 - 25 % en masse |
| F⁻ | 3 - 12 % en masse |
| R₂O | 3,5 - 16 % en masse |

4. Vitrocéramique à base d'apatite selon les revendications 1 ou 2, caractérisée en ce qu'elle a la composition suivante :
| | |
|---|---|
| SiO₂ | 15 - 40 % en masse |
| Al₂O₃ | 15 - 35 % en masse |
| CaO | 12 - 30 % en masse |
| P₂O₅ | 8 - 25 % en masse |
| F⁻ | 4 - 11 % en masse |
| R₂O | 6 - 10 % en masse |

5. Vitrocéramique à base d'apatite selon les revendications 1 à 4, caractérisée en ce qu'elle contient des additifs en une quantité allant jusqu'à 25 % en masse, seuls ou en mélange, à l'aide desquels on obtient une transparence aux rayons X, de préférence SrO, La₂O₃_{,} ou à l'aide desquels on arrive à une coloration de la vitrocéramique, de préférence FeO, Fe₂O₃, TiO₂.

6. Vitrocéramique à base d'apatite selon les revendications 1 à 5, caractérisée en ce qu'elle a été préparée à partir de verres de départ ayant subi une élaboration à 1450-1600°C et une démixtion.

7. Vitrocéramique à base d'apatite selon les revendications 1 à 6, caractérisée en ce qu'elle a été préparée sur un intervalle de températures de 700 à 1150°C, par un traitement thermique des verres de départ ayant subi une démixtion.

8. Vitrocéramique à base d'apatite selon les revendications 1 à 7, caractérisée en ce que les cristaux d'apatite ont de préférence une grosseur de 0,2 à 3 µm.

9. Vitrocéramique à base d'apatite selon les revendications 1 à 8, caractérisée en ce que la phase cristalline principale d'apatite est représentée par la fluorapatite et/ou par l'hydroxylapatite et/ou par un mélange de fluorapatite et d'hydroxylapatite.

10. Vitrocéramique à base d'apatite selon les revendications 1 à 9, caractérisée en ce que la phase apatite contient des ions aluminium et/ou des ions sodium et/ou des ions potassium et/ou des ions strontium.

11. Utilisation de la vitrocéramique à base d'apatite selon les revendications 1 à 10 comme constituant minéral de ciments de verre-ionomère.
